# EUROPEAN PATENT APPLICATION

(11) **EP 1 782 795 A1**
(43) Date of publication of application: **09.05.2007**
(21) Application number: 06425750.4
(22) Date of filing: 31.10.2006
(51) Int. Cl.: A61K 9/00, A61F 9/00

(54) **Ophthalmic gel made up of triamcinolone acetonide and a polyacrylic polymer**

(30) Priority: 04.11.2005 IT RM20050547
(71) Applicant: Sooft Italia SRL, 63025 Montegiorgio AP (IT)
(72) Inventor: Stagni, Marcello, 95127 Catania (CT) (IT); Cavallo, Giovanni, 00122 Roma (IT); Biondi, Enrico, 63025 Montegiorgio (AP) (IT); Iaselli, Vincenzo, 00054 Roma (IT); Sodo, Eugenio, 00125 Roma (IT)
(74) Representative: Sarpi, Maurizio

(57) **Abstract**

For the preparation of ophthalmic gels containing triamcinolone acetonide as active principle, use is envisaged of gelifying agents, such as homopolymers of acrylic acid cross-linked with an alkyl sucrose or with an alkyl pentaerythritol (carbopol - carbomer).

The triamcinolone acetonide gel obtained can be used as contrast agent of the vitreous body in ophthalmic surgery, as medicament for treatment of ocular pathologies with an allergic and/or inflammatory base, and for treatment of diabetic macular oedema.

## Description

The present invention relates to the pharmaceutical sector and more in particular to the use of gelifying agents, such as homopolymers of acrylic acid cross-linked with an alkyl sucrose or with a pentaerythritol (carbopol-carbomer) for the preparation of ophthalmic gels containing triamcinolone acetonide as active principle.

As is known, triamcinolone acetonide is a glucocorticoid, the crystals of which are insoluble in water (melting point: 292-294°C).

It is currently used as active principle in injectable suspensions, such as KENALOG^{®} (Bristol Myers Squibb), marketed in Italy under the name KENACORT^{®}.

KENALOG^{®} is available in two distinct concentrations:
a) 10 mg/ml, suitable for intradermal, intra-articular and intrabursal injections;
b) 40 mg/ml, for intramuscular and intra-articular injections.

In the ophthalmic field (which is the field of application of the present invention), the specific indication for this glucocorticoid is the one approved for serious chronic disorders with an allergic and inflammatory base, such as: ocular herpes zoster; iritis; iridocyclitis; chorioretinitis; diffused uveitis and posterior choroiditis; optical neuritis; sympathetic ophthalmia; and inflammation of the anterior sector.

The aforesaid molecule has found valid use also in the treatment of diabetic macular oedema and in this case it is injected directly into the vitreous body.

Starting from the year 2000, moreover, triamcinolone acetonide has been used in ophthalmic surgery and more precisely as aid in vitrectomy, i.e., in the removal of the vitreous or fibrovascular membrane from the retina in subjects affected by proliferative vitreoretinopathy (PVR) or by proliferative diabetic retinopathy (PDR). In fact, the intra-ocular injection of triamcinolone acetonide enables visualization of the vitreous body, facilitating separation of the vitreum from the retina. Likewise, the injection of triamcinolone acetonide in the anterior chamber can facilitate visualization and subsequent removal of the vitreous body also during cataract surgery.

Use in ophthalmic surgery of triamcinolone acetonide creates, however, a number of difficulties.

In fact, administration of the product by means of an intra-ocular injection is problematical, since, on account of its high insolubility in water, the product tends to precipitate over time.

In the literature, in support of what has been said, different techniques and procedures are reported, described by various authors [1, 2, 3, 4], according to which the commercial product KENALOG^{®}, (the composition is described in Table 1 below) must be treated to release it from the stabilizing/antibacterial and thickening agents, and then triamcinolone acetonide is re-dispersed in an appropriate volume of balanced salt solution (BSS).

**Table 1**

| Component | Concentration | Function |
|---|---|---|
| Triamcinolone acetonide | 4.0% (40 mg/ml) | Active principle |
| Sodium carboxymethylcellulose | 0.75% | Suspending agent |
| Polysorbate 80 | 0.04% | Surfactant |
| Sodium chloride | q.s. for isotonicity | Isotonicity-regulating agent |
| Benzyl alcohol | 0.99 | Preserving agent |
| NaOH / HCl | q.s. for pH 5.0-7.5 | pH-regulating agent |
| Water for injection | to reach 100 g | |

A volume comprised between 0.1 and 0.5 ml of this dispersion of triamcinolone acetonide is injected with a needle, of a diameter comprised between 23 and 27, at the centre of the vitreous cavity.

The granules of triamcinolone are entrapped in the structure of the vitreum. In this manner, the cortical posterior part appears as a white gel, and on the temporal vein of the retina there appears a fracture of the posterior hyaloid cortex.

Thanks to the visualization of this fracture of the hyaloid cortex, it becomes more convenient to insert, along its edge, a vitrectomy probe. Also after aspiration of the vitreum, the possible presence of residues of the vitreous cortex can be visualized thanks to the presence of triamcinolone-acetonide crystals.

The procedures, of which a brief description has been provided above, are complicated and are not easily reproducible. In fact, as has already been mentioned previously, on account of its insolubility in water, the product tends obviously to reprecipitate [5, 6] causing the drawbacks that can be summarized as follows:
- administration is necessary within a short time to prevent precipitation of the active principle;
- precipitation of the crystals of triamcinolone acetonide may obstruct the needle necessary for intra-ocular administration;
- the amount of active principle administrated is not always constant;
- traces of antibacterial products and/or preserving agents may be present;
- the asepsis of the suspension is not guaranteed.

In order to solve at least in part the above difficulty of administration, various formulations have been proposed, containing:
- carboxymethylcellulose (KENACORT^{®}),
- polyvinylpyrrolidone [6],
- methylcellulose[7],
- microparticles[8],
   which, however, do not solve completely all the technical problems highlighted above.

Unlike the known art, the present invention is able to overcome simultaneously all the technical drawbacks referred to above in particular envisaging the use of gelifying agents or rather of homopolymers of acrylic acid cross-linked with an alkyl sucrose or with an alkyl pentaerythritol (carbopol - carbomer).

Carbopol forms a triamcinolone acetonide gel, having an excellent flow capacity combined with a high suspending power (low or zero thixotropy, high yield value). After three months, the gels do not present any precipitate.

A further characteristic of the invention is represented by the use of trehalose as regulator of the osmolarity of said ophthalmic gel. Given its characteristics of stability at even low values of pH and above all at high temperatures, trehalose enables steam sterilization of the gels containing carbopol and triamcinolone acetonide, without impairing the stability and rheological characteristics of the gel.

The characteristics of the invention will emerge more clearly from the ensuing description with reference to the attached plates of drawings, in which:
- Figure 1 is a brief summary of the different types of rheological behaviours;
- Figure 2 shows the difference between two curves of viscosity of a fluid, the first obtained under increasing stress and the second obtained with a stress that decreases in time; and
- Figures 3 and 4 are photographic illustrations of the different behaviours of the product Kenacort (triamcinolone acetonide) and of the triamcinolone gel forming the subject of the present invention, injected in the vitreum of a rabbit affected by induced diabetic retinopathy.

The polymers identified by the name "carbopol" (carbomer) in water tend to swell when they are brought to a pH comprised between 5 and 7; since the pH of these polymers is 6 +/- 0.5, the carboxylic groups of the polymeric backbone ionize causing repulsion between the negative charges with subsequent swelling and gelification of the polymer.

A peculiar characteristic of the gels produced from carbopol is that of having:
- an excellent suspending power, defined in rheology as "yield value"; and
- a good flow capacity; they are only slightly thixotropic or not thixotropic at all.

As may be seen in Figure 1,
a) when Newtonian fluids are subjected to a SHEAR STRESS, they decrease their viscosity in a way proportional to said force; in this case, if the shear stress is plotted on a graph (axis of the ordinate) as a function of the shear rate (axis of the abscissa), a straight line passing through the origin is obtained;
b) when fluids with non-Newtonian behaviour are subjected to a SHEAR STRESS, they decrease their viscosity in a way not proportional to said stress; in this case, if the shear stress is plotted on a graph (axis of the ordinate) as a function of the shear rate (axis of the abscissa), two types of curve are obtained:
   - one passing through the origin; and
   - the other intercepting the axis of the ordinate in one point; the value of the shear stress in this point is called YIELD VALUE (suspending power).

Fluids subjected to a shear stress decrease their viscosity in various ways, and, when the stress is no longer applied, they resume the initial viscosity in a more or less long period.

The difference between the two viscosity curves, namely, the one obtained under increasing stress and the one obtained with a stress that decreases in time, is referred to as "thixotropy" (see Figure 2);
- fluids with a low flow capacity have a high thixotropy; and
- fluids with a high flow capacity have a low or zero thixotropy.

Gels formed by carbopols present the following characteristics:
a) they have an ELLIS-type behaviour and an excellent suspending power: the insoluble product entrapped in the gel does not precipitate;
b) they are only slightly thixotropic or not thixotropic at all: they are hence pumpable.

From the rheological considerations referred to above, the formulations illustrated in the ensuing Tables A, B, C, D, E and F were derived. Given in said tables are also the viscosity value, the yield value and the value of thixotropy using the Herschel-Bulkey and Casson mathematical models, respectively.

**Formulation A**

| Ingredient | Concentration | Function |
|---|---|---|
| Triamcinolone acetonide | 1.0% w/w | Active principle |
| Carbopol 980 NF | 0.1% w/w | Gelifying agent |
| NaOH | to reach 6.5-7 | pH-regulating agent |
| Water for injectables | to reach 100 g | |
| Viscosity | 5000-10000 mPa.s | |
| Yield value | 5000-15000 mPa | |
| Thixotropy | -30-0 mPa/s | Resumes the initial structure |
| Isotonicity | **???**mOs | Highly hypotonic |
| Presence of precipitate at | 6 months | No |

**Formulation B**

| Ingredient | Concentration | Function |
|---|---|---|
| Triamcinolone acetonide | 2.0% w/w | Active principle |
| Carbopol 980 NF | 0.1% w/w | Gelifying agent |
| NaOH | to reach 6.5-7 | PH-regulating agent |
| Water for injectables | to reach 100 g | |
| Viscosity | 5000-10000 mPa.s | |
| Yield value | 5000-15000 mPa | |
| Thixotropy | -20-0 mPa/s | Resumes the initial structure |
| Isotonicity | Mos | Highly hypotonic |
| Presence of precipitate at | 6 months | No |

**Formulation C**

| Ingredient | Concentration | Function |
|---|---|---|
| Triamcinolone acetonide | 3.0% (30 mg/ml) | Active principle |
| Carbopol 980 NF | 0.15% | Gelifying agent |
| NaOH | to reach 6.5-7 | PH-regulating agent |
| Water for injectables | to reach 100 g | |
| Viscosity | 8000-15000 mPa.s | |
| Yield value | 10000-15000 mPa | |
| Thixotropy | -10 - 0 mPa/s | Resumes the initial structure |
| Isotonicity | Mos | Highly hypotonic |
| Presence of precipitate at | 6 months | No |

**Formulation D**

| Ingredient | Concentration | Function |
|---|---|---|
| Triamcinolone acetonide | 4.0% (40 mg/ml) | Active principle |
| Carbopol 980 NF | 0.2% | Gelifying agent |
| NaOH | to reach 6.5-7 | PH-regulating agent |
| Water for injectables | to reach 100 g | |
| Viscosity | 20000- 30000 mPa.s | |
| Yield value | 15 000 -25000 mPa | |
| Thixotropy | -5- 0 mPa/s | Resumes the initial structure |
| Isotonicity | Mos | Highly hypotonic |
| Presence of precipitate at | 6 months | No |

**Formulation E**

| Ingredient | Concentration | Function |
|---|---|---|
| Triamcinolone acetonide | 6.0%(60 mg/ml) | Active principle |
| Carbopol 980 NF | 0.25% | Gelifying agent |
| NaOH | to reach 6.5-7 | pH-regulating agent |
| Water for injectables | to reach 100 g | |
| Viscosity | 30000-40000 mPa.s | |
| Yield value | 28000-38000 mPa | |
| Thixotropy | 1mPa/s | Modifies initial structure only slightly |
| Isotonicity | MOs | Highly hypotonic |
| Presence of precipitate at | 6 months | No |

**Formulation F**

| Ingredient | Concentration | Function |
|---|---|---|
| Triamcinolone acetonide | 8.0%(80 mg/ml) | Active principle |
| Carbopol 980 NF | 0.3% | Gelifying agent |
| NaOH | to reach 6.5-7 | PH-regulating agent |
| Water for injectables | to reach 100 g | |
| Viscosity | 40000-50000 mPa.s | |
| Yield value | 3500-45000 mPa | |
| Thixotropy | 10 mPa/s | Modifies initial structure |
| Isotonicity | MOs | Highly hypotonic |
| Presence of precipitate at | 6 months | A few crystals |

(The values of viscosity were evaluated using a plate rheometer manufactured by TA Instruments 2000 with 60-mm acrylic plate at a controlled temperature of 25°C).

### Preparation of ISOTONIC ophthalmic gels

The formulations described previously are all hypotonic.

Advantageously, according to the present invention, isotonicity was reached by adding an appropriate amount of a disaccharide: in particular, trehalose was chosen on account of its compatibility with the gelifying agent carbopol and on account of its chemico-physical characteristics, as will be described hereinafter. Given hereinafter are the compositions and characteristics of the isotonic gels obtained starting from the formulations B, C, D and E described previously.

**ISOTONIC gel B**

| Ingredient | Concentration | Function |
|---|---|---|
| Triamcinolone acetonide | 2.0% w/w | Active principle |
| Carbopol 980 NF | 0.1% w/w | Gelifying agent |
| Trehalose | 9% w/w | Osmolarity-regulating agent |
| NaOH | to reach 6.5-7 | PH-regulating agent |
| Water for injectables | to reach 100 g | |
| Viscosity | 7000-12000 mPa.s | |
| Yield value | 7000-17000 mPa | |
| Thixotropy | -20-0 mPa/s | Resumes the initial structure |
| Isotonicity | 280-300 mOs | Isotonic gel |
| Presence of precipitate at | 6 months | No |

**ISOTONIC gel C**

| Ingredient | Concentration | Function |
|---|---|---|
| Triamcinolone acetonide | 3.0% w/w | Active principle |
| Carbopol 980 NF | 0.15% w/w | Gelifying agent |
| Trehalose | 9% w/w | Osmolarity-regulating agent |
| NaOH | To reach 6.5-7 | pH-regulating agent |
| Water for injectables | To reach 100 g | |
| Viscosity | 10000-17000 mPa.s | |
| Yield value | 13000-18000 mPa | |
| Thixotropy | -10 - 0 mPa/s | Resumes the initial structure |
| Isotonicity | 280-300 mOs | Isotonic gel |
| Presence of precipitate at | 6 months | No |

**ISOTONIC gel D**

| Ingredient | Concentration | Function |
|---|---|---|
| Triamcinolone acetonide | 4.0% w/w | Active principle |
| Carbopol 980 NF | 0.2% w/w | Gelifying agent |
| Trehalose | 9% w/w | Osmolarity-regulating agent |
| NaOH | to reach 6.5-7 | pH-regulating agent |
| Water for injectables | to reach 100 g | |
| Viscosity | 22000- 32000 mPa.s | |
| Yield value | 17000 -26000 mPa | |
| Thixotropy | -5- 0 mPa/s | Resumes the initial structure |
| Isotonicity | 280-300 mOs | Isotonic gel |
| Presence of precipitate at | 6 months | No |

**ISOTONIC gel E**

| Ingredient | Concentration | Function |
|---|---|---|
| Triamcinolone acetonide | 6.0% w/w | Active principle |
| Carbopol 980 NF | 0.25% w/w | Gelifying agent |
| Trehalose | 9% w/w | Osmolarity regulator |
| NaOH | to reach 6.5-7 | pH-regulating agent |
| Water for injectables | to reach 100 g | |
| Viscosity | 32000-42000 mPa.s | |
| Yield value | 30000-40000 mPa | |
| Thixotropy | 2-0 mPa/s | Modifies initial structure only slightly |
| Isotonicity | 280-300 mOs | Isotonic gel |
| Presence of precipitate at | 6 months | No |

### Choice of the ISOTONICITY-regulating agent

As mentioned previously, the choice of trehalose [9, 10, 11] as regulator of the isotonicity of the gel was made on the basis of the considerations referred to below.

### a - Compatibility with the gelifying agent Carbopol

- The isotonicity regulator must not interfere with the gelifying properties of carbopol.
- The isotonicity regulator must not modify the viscosity, thixotropy, and yield value of the gel.

As a demonstration of what has been said above, by way of comparison, the gels B and E were rendered isotonic with the addition of sodium chloride instead of trehalose, with the result that all the gels completely lost their rheological characteristics, as emerges from the tables appearing below.

**Gel B rendered isotonic with sodium chloride**

| Ingredient | Concentration | Function |
|---|---|---|
| Triamcinolone acetonide | 2.0% w/w | Active principle |
| Carbopol 980 NF | 0.1% w/w | Gelifying agent |
| Sodium chloride | 0.85% w/w | Osmolarity regulator |
| NaOH | To reach 6.5-7 | pH-regulating agent |
| Water for injectables | To reach 100 g | |
| Viscosity | 1000 mPa.s | |
| Yield value | ------- | |
| Thixotropy | +250 mPa/s | The structure of the gel is destroyed |
| Isotonicity | 300 mOs | Isotonic gel |
| Presence of precipitate at | 20 minutes | Yes |

**Gel E rendered isotonic with sodium chloride**

| Ingredient | Concentration | Function |
|---|---|---|
| Triamcinolone acetonide | 6.0% w/w | Active principle |
| Carbopol 980 NF | 0.25% w/w | Gelifying agent |
| Sodium chloride | 0.85% w/w | Osmolarity regulator |
| NaOH | To reach 6.5-7 | pH-regulating agent |
| Water for injectables | To reach 100 g | |
| Viscosity | 1000 mPa.s | |
| Yield value | ------- | |
| Thixotropy | +250 mPa/s | The structure of the gel is destroyed |
| Isotonicity | 300 mOs | Isotonic gel |
| Presence of precipitate at | 20 minutes | Yes |

### b- Chemico-physical characteristics of the disaccharide

Trehalose is a sugar (disaccharide) with characteristics different from other disaccharides with the same molecular weight.

| Properties | Trehalose | Maltose | Sucrose |
|---|---|---|---|
| Molecular weight | 342.31 | 342.31 | 342.31 |
| Melting point | 97.0 °C | 102.5°C | 160-168°C |
| Temp. Vitreous transition | 79°C | 43°C | 52°C |
| Reducing sugar | No | Yes | No |
| Stability at acid pH <3 | Yes | No | No |
| Stability at high temperatures | Yes | No | No |
| Maillard reaction | No | Yes | Yes |
| Stabilization of the proteins | Yes (+++) | No | Yes (+) |

### Sterilization of ophthalmic gels

The choice of trehalose, given its stability also at high temperatures, enables steam sterilization of the gel while maintaining the rheological characteristics thereof, eliminating the problem of traces of the preserving agents referred to above as most frequent cause of ocular toxicity.

Initially, a mixture of triamcinolone acetonide at 2% and trehalose at 9% was subjected to steam sterilization (at the pressure of 1 atm at 121°C for 25 minutes). The analyses of stability after sterilization were made following the HPLC method indicated by the United States pharmacopoeia [12].

The results are summarized in the table below.

| | Non-sterilized | Sterilized |
|---|---|---|
| Triamcinolone acetonide | 2% (20 mg/ml) | 1.9% (19 mg/ml) |
| Colour of mixture | White | White |

The gels of triamcinolone and carbopol and trehalose were sterilized with the same modalities.

The tables reproduced below summarize the results obtained.

**ISOTONIC gel B after steam sterilization**

| Ingredient | | Characteristics after sterilization |
|---|---|---|
| Triamcinolone acetonide | 2.0% w/w | Unvaried |
| Carbopol 980 NF | 0.1% w/w | |
| Trehalose | 9.0% w/w | |
| NaOH | to reach 6.5-7 | |
| Water for injectables | to reach 100 g | |
| Viscosity | 6000-1000 mPa.s | Substantially unvaried |
| Yield value | 6000-16000 mPa | Substantially unvaried |
| Thixotropy | -18 mPa/s | Substantially unvaried |
| Isotonicity | 280- 300 MOs. | Unvaried |
| Presence of precipitate at | 3 months | No |
| Colour | White | Unvaried |

**ISOTONIC gel C after steam sterilization**

| Ingredient | | Characteristics after sterilization |
|---|---|---|
| Triamcinolone acetonide | 3.0% w/w | Unvaried |
| Carbopol 980 NF | 0.15% w/w | |
| Trehalose | 9.0% w/w | |
| NaOH | to reach 6.5-7 | |
| Water for injectables | to reach 100 g | |
| Viscosity | 9000-16000 mPa.s | Substantially unvaried |
| Yield value | 12000-17000 mPa | Substantially unvaried |
| Thixotropy | -9 mPa/s | Substantially unvaried |
| Isotonicity | 280-300 mOs | Unvaried |
| Presence of precipitate at | 3 months | No |
| Colour | White | Unvaried |

**ISOTONIC gel D after steam sterilization**

| Ingredient | | Characteristics after sterilization |
|---|---|---|
| Triamcinolone acetonide | 4.0% w/w | Unvaried |
| Carbopol 980 NF | 0.2% w/w | |
| Trehalose | 9.0% w/w | |
| NaOH | to reach 6.5-7 | |
| Water for injectables | to reach 100 g | |
| Viscosity | 21000-31000 mPa.s | Substantially unvaried |
| Yield value | 16000-26000 mPa | Substantially unvaried |
| Thixotropy | -2 mPa/s | Substantially unvaried |
| Isotonicity | 280-300 mOs | Unvaried |
| Presence of precipitate at | 3 months | No |
| Colour | White | Unvaried |

**ISOTONIC gel E after steam sterilization**

| Ingredient | | Characteristics after sterilization |
|---|---|---|
| Triamcinolone acetonide | 6.0% w/w | Unvaried |
| Carbopol 980 NF | 0.25% w/w | |
| Trehalose | 9.0% w/w | |
| NaOH | to reach 6.5-7 | |
| Water for injectables | to reach 100 g | |
| Viscosity | 31000-41000 mPa.s | Substantially unvaried |
| Yield value | 29000-39000 mPa | Substantially unvaried |
| Thixotropy | -0 mPa/s | Substantially unvaried |
| Isotonicity | 280-300 mOs | Unvaried |
| Presence of precipitate at | 3 months | No |
| Colour | White | Unvaried |

### Evaluation of carbopol triamcinolone gel vs. Kenacort

As has been said previously in the introduction, triamcinolone acetonide is used in ophthalmic surgery as aid in vitrectomy, i.e., in removal of the vitreous or fibrovascular membrane from the retina in patients affected by proliferative vitreoretinopathy (PVR) or by proliferative diabetic vitreoretinopathy (PDR).

Consequently, it has appeared expedient to us to compare the new triamcinolone acetonide gel with Kenacort in a model of diabetic retinopathy induced in rabbits which envisaged an intravitreal injection.

Given hereinafter are the salient points of the experiment conducted.
~ All the rabbits were affected by experimentally induced diabetes, and in all of them a diabetic retinopathy developed.
~ After of the presence of diabetic retinopathy had been ascertained via fluoroangiography, triamcinolone acetonide (Kenacort) was injected in the vitreum of the right eye, and sterile isotonic carbopol triamcinolone gel was injected in the left eye at the doses of 2 mg/day, an amount used for therapeutic purposes in humans.
~ One rabbit was sacrificed every 15 days from intravitreal injection, after subjecting it to measurement of the intraocular pressure (IOP) and to retinal fluoroangiography.
~ The IOP was always found within the normal limits, to be precise within the range of values 8 to 16 mmHg (normal values for rabbits; for humans, said values range between 8 and 20 mmHg). Hence, neither of the two products, namely, Kenacort and the new 2%-triamcinolone gel, had a significant effect on the IOP in the short term.
~ Via fluoroangiography is was possible to ascertain that the diabetic retinopathy had sensibly regressed, i.e., there was a reduction in the number of haemorrhages and retinal oedemas, which is a sign that both of the two products, namely, Kenacort and the new 2%-triamcinolone gel, are active against diabetic retinopathy.
~ With fluoroangiography the state of the vitreous gel was moreover checked and it was noted that the new triamcinolone acetonide gel administrated with 27-30-diameter needles diffused homogeneously in the vitreum, rendering it well visible and at the same time rendering the retina explorable (see triamcinolone gel photo).
~ Triamcinolone acetonide (Kenacort) does not diffuse; it forms, instead, a compact mass that floats in the vitreum without opacizing it uniformly (see Kenacort photo)
∼ Neither of the products (Kenacort or triamcinolone gel) caused onset of cataract in the short term.

### Evaluation of BIOCOMPATIBILITY of triamcinolone acetonide gel

The sterile triamcinolone acetonide gel B (the composition is given hereinafter) was evaluated for ocular biocompatibility. In particular, the following tests were carried out:
- citotoxicity test by direct contact;
- delayed-hypersensitivity test;
- ocular-irritability test;
- AMES (mutagenicity) test

**STERILE ISOTONIC gel B**

| Ingredient | |
|---|---|
| Triamcinolone acetonide | 2.0% w/w |
| Carbopol 980 NF | 0.1% w/w |
| Trehalose | 9.0% w/w |
| NaOH | to reach 6.5-7 |
| Water for injectables | to reach 100 g |

The results of the study conducted at research laboratories have shown how the isotonic and sterile gel B is biocompatible after vitreous administration, as emerges from what follows.
- citotoxicity test by direct contact
   The product did not prove cytotoxic
- delayed-hypersensitivity test
   The product did not prove sensitizing
- ocular-irritability test
   The product did not prove irritating
- AMES (mutagenicity) test
   The product did not prove mutagenic

### SINGLE-USE ADMINISTRATION WITH PREFILLED SYRINGE

The characteristics of triamcinolone acetonide gel, which forms the subject of the present patent, have enabled administration of the active principle in a pre-defined single dose in a syringe.

In fact the possibility of having a gel with
- good suspending power,
- good flow capacity: in practical terms good "pumpability" at an industrial level,
- facility of INTRA-OCULAR administration, and
- good stability to sterilization due to its peculiar composition,
   has enabled implemention at the level of industrial production of filling of 1-ml glass syringes containing from 0.2 to 1 ml of gel.

Glass syringes were chosen in so far as they enable, once filled with the desired amount of product, steam sterilization at 121°C for 20 minutes.

### Sterilization of glass syringes pre-filled with triamcinolone acetonide gel

In order to supply possible users with a product ready for use, already dosed, and with the maximum level of asepsis, glass syringes were used, which enable filling and subsequent sterilization.

The final sterilization of the product inside the syringe enables:
- simplification of the process of production; and
- increase in the asepsis of the end product.

In this connection, the stability of the gel according to the invention was evaluated, after being introduced in the syringes and subsequently sterilized.

Given hereinafter are, in particular, the data of stability of the 2% triamcinolone acetonide gel prepared in syringes (1 ml):

**ISOTONIC gel B after steam sterilization in syringe**

| Ingredient | Concentration | Characteristics after sterilization |
|---|---|---|
| Triamcinolone acetonide | 2.0% w/w | Unvaried |
| Presence of precipitate at | 3 months | No |
| Colour | White | Unvaried |

### Accelerated stability tests on syringes filled with 2% triamcinolone acetonide gel

Syringes with 1 ml of 2% triamcinolone acetonide gel were sterilized (121°C for 20 minutes at the pressure of 1 atm) and kept in a stove at 40°C according to the following scheme:

| | Number of syringes | Triamcinolone mg/ml * | Colour | Presence of precipitate |
|---|---|---|---|---|
| Month | | | | |
| 0 | 3 | 20 mg/ml | White | No |
| 1 | 3 | 19.5 mg/ml | White | No |
| 2 | 3 | 19.5 mg/ml | White | No |
| 3 | 3 | | | |

| | | | | |
|---|---|---|---|---|
| * average of 3 values | | | | |

**ISOTONIC gel B after steam sterilization**

| Ingredient | Concentration | Characteristics after sterilization |
|---|---|---|
| Triamcinolone acetonide | 2.0% (20 mg/ml) | Unvaried |
| Carbopol 980 NF | 0.1% | |
| Trehalose | 9.0% | |
| NaOH | to reach 6.5-7 | |
| Water for injectables | to reach 100 g | |
| Viscosity | 6000-11000 mPa.s | Substantially unvaried |
| Yield value | 6000-16000 mPa | Substantially unvaried |
| Thixotropy | -18 mPa/s | Substantially unvaried |
| Isotonicity | 280- 300 mOs | Unvaried |
| Presence of precipitate at | 3 months | No |
| Colour | White | Unvaried |

**ISOTONIC gel C after steam sterilization**

| Ingredient | Concentration | Characteristics after sterilization |
|---|---|---|
| Triamcinolone acetonide | 3.0% (30 mg/ml) | Unvaried |
| Carbopol 980 NF | 0.15% | |
| Trehalose | 9.0% | |
| NaOH | To reach 6.5-7 | |
| Water for injectables | To reach 100 g | |
| Viscosity | 9000-16000 mPa.s | Substantially unvaried |
| Yield value | 12000-17000 mPa | Substantially unvaried |
| Thixotropy | -9 mPa/s | Substantially unvaried |
| Isotonicity | 280-300 mOs | Unvaried |
| Presence of precipitate at | 3 months | No |
| Colour | White | Unvaried |

**ISOTONIC gel D after steam sterilization**

| Ingredient | Concentration | Characteristics after sterilization |
|---|---|---|
| Triamcinolone acetonide | 4.0% (40 mg/ml) | Unvaried |
| Carbopol 980 NF | 0.2% | |
| Trehalose | 9.0% | |
| NaOH | to reach 6.5-7 | |
| Water for injectables | to reach 100 g | |
| Viscosity | 21000-31000 mPa.s | Substantially unvaried |
| Yield value | 16000-26000 mPa | Substantially unvaried |
| Thixotropy | -2 mPa/s | Substantially unvaried |
| Isotonicity | 280-300 mOs | Unvaried |
| Presence of precipitate at | 3 months | No |
| Colour | White | Unvaried |

**ISOTONIC gel E after steam sterilization**

| Ingredient | Concentration | Characteristics after sterilization |
|---|---|---|
| Triamcinolone acetonide | 6.0% (60 mg/ml) | Unvaried |
| Carbopol 980 NF | 0.25% | |
| Trehalose | 9.0% | |
| NaOH | To reach 6.5-7 | |
| Water for injectables | To reach 100 g | |
| Viscosity | 31000-41000 mPa.s | Substantially unvaried |
| Yield value | 29000-39000 mPa | Substantially unvaried |
| Thixotropy | -0 mPa/s | Substantially unvaried |
| Isotonicity | 280-300 mOs | Unvaried |
| Presence of precipitate at | 3 months | No |
| Colour | White | Unvaried |

### Sterilizable biocompatible isotonic gel

The gel B of triamcinolone acetonide, carbopol 980NF and trehalose, proved biocompatible from the tests conducted at the BIOLABO laboratories, as emerges from what follows.
- citotoxicity test by direct contact
   The product did not prove cytotoxic
- delayed-hypersensitivity test
   The product did not prove sensitizing
- ocular-irritability test
   The product did not prove irritating
- AMES (mutagenicity) test
   The product did not prove mutagenic

So far preferred compositions of the triamcinolone gel in which Carbopol 980 NF is used have been described. It is, however, pointed out that the same results can be obtained, by applying slight modifications that are evident to a person skilled in the branch, using Carbopol 981 and without departing from the scope of protection of the present industrial patent right, as defined in the ensuing claims.

### REFERENCES

[1] Peyman GA, Cheema R, Conway MD, Fang T. - Triamcinolone acetomide as an aid to visualization of the eye vitreous and the posterior hyaloid during pars plana vitrectomy - Retina 20: 554-555, 2000
[2] Kumagai K. Introduction of a new method for the preparation of triamcinolone acetonide solution as an aid to visualization of vitreous and the posterior hyaloid during pars plana vitrectomy -Retina 23: 881-882,2003
[3] Nishimura A., Isolating triamcinolone acetonide particles for intravitreal use with a porous membrane filter - Retina 23: 777-779 2003
[4] Burk Scott E., Da Mata P. Andrea, Visualizing vitreous using kenalog suspension J.Cataract refract. Surg. 29:645-651 2003
[5] Lemley C.A., Saperstein D.A. Injection quantity decreases with delivery delay in an in vitro model of intravitreal triamcinolone acetonide injection Poster board number 1456//B225 ARVO 2005
[6] Rajni J, Castillo EJ, Han WW - Triamcinolone acetonide and anecortave acetate formulations for injection - Patent US 2005/0065137 A1 (= WO 2005032510), March 24, 2005 Alcon Ltd.
[7] Kube T. Sutter M. Triamcinolone acetonide gel for intravitreal use Poster board number 485/B459 ARVO 2005
[8] Wanczinski B.J. Silva A.A. Triamcinolone acetonide-loaded pellets obtained from PLGA microparticles for intravenous controlled drug release Poster board number 491/B465 ARVO 2005
[9] Iglesias H.A., Chirife T., Buera M.P. Adsorption isotherm of amorphous trehalose J. Sci. Fd. Agric. 75: 183-186 (1997)
[10] O'Brien J. Stability of trehalose, sucrose, and glucose to non enzymatic browning in model systems J.Fd.Sci &1:679-682 (1996)
[11] Crowe J.H. Crowe L.M.,Carpenter J.F., Interactions of sugars with membranes -Biochim. Biophys. Acta 947:367-384 (1988)
[12] The United States Pharmacopoeia 19th rev 1975 p.566

## Claims

1. A formulation comprising triamcinolone acetonide and homopolymers of acrylic acid cross-linked with an alkyl sucrose or with an alkylpentaerythritol (carbopol - carbomer).

2. The formulation as per Claim 1, **characterized in that** the concentration of triamcinolone acetonide is between 0.05 and 16% w/w.

3. The formulation according to Claim 1,
**characterized in that** the concentration of triamcinolone acetonide is between 1 and 8% w/w.

4. The formulation according to Claim 1,
**characterized in that** the concentration of carbopol is between 0.05 and 0.8%.

5. The formulation according to Claim 1,
**characterized in that** the concentration of carbopol is between 0.1 and 0.3%.

6. The formulation according to any one of the preceding claims, **characterized in that** the carbopol is Carbopol 980 NF.

7. The formulation according to any one of the preceding claims, **characterized in that** it is hypotonic.

8. A composition comprising any one of the formulations of Claims 1 to 7 and trehalose.

9. The composition according to Claim 8,
**characterized in that** the concentration of trehalose is between 5 and 11%.

10. The composition according to Claim 9,
**characterized in that** it is in the form of a gel.

11. The composition according to Claim 10,
**characterized in that** it is isotonic.

12. A composition comprising:
| | |
|---|---|
| Triamcinolone acetonide: | 2.0% (20 mg/ml) |
| Carbopol 980 NF: | 0.1% |
| Trehalose: | 9.2% |
| NaOH: | to reach 6.5-7 |
| Water for injectables: | to reach 100 g |

13. A composition comprising:
| | |
|---|---|
| Triamcinolone acetonide: | 3.0% (30 mg/ml) |
| Carbopol 980 NF: | 0.15% |
| Trehalose: | 9.2% |
| NaOH: | to reach 6.5-7 |
| Water for injectables: | to reach 100 g |

14. A composition comprising:
| | |
|---|---|
| Triamcinolone acetonide: | 4.0% (40 mg/ml) |
| Carbopol 980 NF: | 0.2% |
| Trehalose: | 9.2% |
| NaOH: | to reach 6.5-7 |
| Water for injectables: | to reach 100 g |

15. The composition according to Claims 8 to 14, **characterized in that** it is subjected to steam sterilization.

16. Use of the composition according to any one of the preceding claims for preparation of a medicament in the form of a gel for ophthalmic use.

17. Use of the medicament according to Claim 16 as contrast agent of the vitreous body in ophthalmic surgery.

18. Use of the medicament according to Claim 16 for treatment of ocular pathologies with an allergic and/or inflammatory base.

19. Use of the medicament according to Claim 16 for treatment of diabetic macular oedema.

20. A medicament according to Claim 16,
**characterized in that** it is administered by means of a syringe in a pre-defined single dose by means of a pre-filled glass syringe.

21. The medicament according to the preceding claim, **characterized in that** said pre-filled glass syringe is subjected to steam sterilization.
